# FASCICULE DE BREVET EUROPEEN

(11) **EP 2 879 657 B1**
(45) Date de publication et mention de la délivrance du brevet: **13.11.2019**
(21) Numéro de dépôt: 13765519.7
(22) Date de dépôt: 02.08.2013
(51) Int. Cl.: A61K 8/63, A61Q 19/06, A61Q 19/08, A61K 31/57, A61K 31/58, A61K 31/585

(54) **NOUVEAUX COMPOSÉS ACTIVATEURS DE L'ACTIVITÉ ENZYMATIQUE DE SIRT1 ET COMPOSITIONS PHARMACEUTIQUES OU COSMÉTIQUES LES COMPRENANT**
NEUE SIRT1 AKTIVATOREN UND PHARMAZEUTISCHE ODER KOSMETISCHE ZUSAMMENSETZUNGEN ENTHALTEND DIESE AKTIVATOREN
NOVEL SIRT1 ACTIVATORS AND PHARMACEUTICAL OR COSMETIC COMPOSITIONS COMPRISING THEM

(30) Priorité: 03.08.2012 FR 1257598
(43) Date de publication de la demande: 10.06.2015
(73) Titulaire: Centre National de la Recherche Scientifique (C.N.R.S.), 75016 Paris (FR)
(72) Inventeur: BAKALA, Johanna, F-75012 Paris (FR); BIGNON, Jérôme, F-91530 Le Val St Germain (FR); LIU, Jian-Miao, F-75013 Paris (FR); GUILLOU, Catherine, F-91190 Gif-sur-Yvette (FR); ACHAB, Maria, Concepcion, F-91190 Gif-sur-Yvette (FR)
(74) Mandataire: Lavoix
(86) Numéro de dépôt international: PCT/IB2013/056349
(87) Numéro de publication internationale: WO 2014/020577

(56) Documents cités:
- FR-A- 1 539 326
- GB-A- 929 272
- US-A- 3 098 082
- US-A1- 2005 245 495
- US-A1- 2010 040 568
- KHUONG-HUU-QUI ET AL: BULLETIN DE LA SOCIETE CHIMIQUE DE FRANCE, SOCIETE FRANCAISE DE CHIMIE. PARIS, FRANCE, vol. 10, 1 janvier 1965 (1965-01-01), pages 3035-3040, XP009170461, ISSN: 0037-8968 cité dans la demande
- D.D. GODSE ET AL: "Transformation of kurchi alkaloids-III", TETRAHEDRON, vol. 19, no. 5, 1 janvier 1963 (1963-01-01), pages 783-788, XP055067283, ISSN: 0040-4020, DOI: 10.1016/S0040-4020(01)99212-9 cité dans la demande
- HUSSON H P: BULLETIN DE LA SOCIETE CHIMIQUE DE FRANCE, SOCIETE FRANCAISE DE CHIMIE. PARIS, FRANCE, vol. 9, 1 janvier 1969 (1969-01-01), pages 3162-3166, XP009170468, ISSN: 0037-8968 cité dans la demande
- JANOT M-M ET AL: "Alcaloides stéroidiques LXVI. Nouveaux alcaloides des écorces de l'holarrhena floribunda (G.Don) Dur. et Schinz: holarrhéline, holadiénine, holaromine et holaline", ANNALES PHARMACEUTIQUES FRANCAISES, MASSON, PARIS, FR, vol. 25, no. 11, 1 janvier 1967 (1967-01-01), pages 733-748, XP009170464, ISSN: 0003-4509 cité dans la demande
- KHUONG-HUU-LAINÉ F ET AL: "Alcaloides stéroidiques, XXXIX. Alcaloides du Malouteia bequaertiana Woods. Mise au point sur le genre Malouetia (Apocynacées) et sur une drogue curarisante du Venezuela, le Guachamacá", ANNALES PHARMACEUTIQUES FRANCAISES, MASSON, PARIS, FR, vol. 23, no. 6, 1 janvier 1965 (1965-01-01), pages 395-409, XP009170465, ISSN: 0003-4509 cité dans la demande
- JANOT M M: BULLETIN DE LA SOCIETE CHIMIQUE DE FRANCE, SOCIETE FRANCAISE DE CHIMIE. PARIS, FRANCE, 1 janvier 1962 (1962-01-01), pages 111-118, XP009170462, ISSN: 0037-8968 cité dans la demande

## Description

La présente invention se rapporte à de nouveaux activateurs de la sirtuine 1 et à leur utilisation dans le domaine cosmétique et/ou pharmaceutique, notamment dermatologique. Plus particulièrement, ces composés sont utilisés dans une composition destinée au soin de la peau, notamment pour lutter contre les signes du vieillissement cutané et/ou une composition pour lutter contre les amas-graisseux sous-cutanés et/ou la cellulite.

Le processus du vieillissement cutané est un mécanisme cellulaire complexe. Les dermatologues font en effet la distinction entre le vieillissement intrinsèque ou chronologique (génétique et hormonal) et le vieillissement extrinsèque provoqué par des facteurs externes comme les rayons UV, le tabac ou la pollution. Plus précisément, le vieillissement résulte de la perte d'équilibre entre la capacité de l'organisme à maintenir son potentiel réparateur des dommages et l'intensité ainsi que la fréquence de ces dommages.

En général, les signes de vieillissement découlent d'une désorganisation de la matrice cellulaire, tant au niveau du derme que de l'épiderme. La structure cellulaire de la peau change progressivement. Les premiers signes sont la perte de fermeté et l'apparition des ridules et des rides d'expression. Avec l'âge, les rides s'accentuent et la peau perd de plus en plus de son élasticité. Tous ces signes perceptibles concernent l'ensemble du corps même si certaines parties telles que le contour des yeux et de la bouche sont nettement plus atteintes.

De plus en plus souvent, la stratégie de développement de nouveaux produits cosmétiques prend en compte, plus que la simple correction des stigmates visibles liés au vieillissement cutané telles les rides par exemple, les événements biologiques qui génèrent ces stigmates.

Inspirées des dernières recherches sur la longévité, des molécules activatrices de sirtuines ont aujourd'hui gagné leur place en cosmétologie.

Les sirtuines sont une famille de déacétylases nucléaires NAD⁺ dépendantes, jouant un rôle important dans la désacétylation des histones. La sirtuine 1 (Sirt1 ; **S**ilent **i**nformation **r**egulator **1**) est la sirtuine la mieux caractérisée en raison de sa forte activité déacétylase impliquée dans un grand nombre de processus métaboliques *(*Haigis MC et al., Mammalian sirtuins-emerging roles in physiology, aging, and calorie restriction. Genes Dev. 2006;20:2911*).* Sirt1 a de nombreuses cibles moléculaires, parmi lesquelles figurent les protéines p53, FoxO, PPARγ, PGC1α, UCP2, LXR et Ku70, régulateurs clés de différentes fonctions cellulaires.

D'une manière générale, cette enzyme a un effet protecteur contre la sénescence métabolique. Il a été montré que les organismes modifiés, caractérisés par une surexpression de Sirt1, présentent un phénotype d'extension de la durée de vie. Ces découvertes relient ainsi Sirt1 au concept de la promotion de la longévité et du ralentissement du vieillissement *(*Ghosh HS. The anti-aging, metabolism potential of SIRT1. Curr Opin Investig Drugs. 2008;9:1095-102 *;* Imai S. Is Sirt1 a miracle bullet for longevity ? Aging Cell. 2007;6:735*).*

Cependant, avec l'âge, on assiste à une diminution de l'expression de Sirt1 cellulaire, d'où la recherche d'activateurs de l'activité et/ou de la synthèse de cette enzyme comme des candidats aux rôles d'agents anti-vieillissement.

Déjà associée au phénomène du vieillissement, Sirt1 contrôle également l'accumulation des réserves adipeuses (Picard F et al. Molecular links between aging and adipose tissue. Int J Obes. 2005;29 Suppl 1:S3). En effet, Sirt1 réprime un régulateur central de l'adipogénèse, PPARγ (**P**eroxisome **P**roliferator-**A**ctivated **R**eceptor γ) et inhibe l'accumulation de lipides dans les adipocytes *(*Picard F et al. Sirt1 promotes fat mobilization in white adipocytes by repressing PPAR-gamma. Nature, 2004;429:771*).* De plus, la désacétylation par Sirt1 d'un facteur de transcription FoxO1 induit la lipolyse via l'augmentation de l'expression de l'adiponectine *(*Qiao L et al. SIRT1 regulates adiponectin gene expression through Foxo1-C/enhancer-binding protein alpha transcriptional complex. J Biol Chem. 2006;281:39915*)* et de la lipase de triglycérides *(*Chakrabarti P et al. SIRT1 controls lipolysis in adipocytes via FOXO1-mediated expression of ATGL. J Lipid Res. 2011;52:1693*).*

Les résultats des études récentes montrent que la synthèse accrue de la protéine Sirt1 due à l'activation *in vivo* de son gène, mime les effets positifs de la restriction calorique indépendamment du régime alimentaire *(*Wood JG et al. Sirtuin activators mimic caloric restriction and delay ageing in metazoans. Nature. 2004;430:686*;* Bardane L et al. SIRT1 transgenic mice show phenotypes resembling calorie restriction. Aging Cell 2007;6:759*).* De plus, il est apparu qu'activer l'axe Sirt1 protège de l'obésité en stimulant l'élimination des graisses (Feige JN et al. Specific SIRT1 activation mimics low energy levels and protects against diet-induced metabolic disorders by enhancing fat oxidation. Cell Metabolism, 2008,-8:347),

L'accès facilité à l'alimentation rapide riche en énergie, l'inactivité physique et le vieillissement de la population sont des facteurs qui ont été identifiés pour expliquer la croissance de la population obèse. Puisque régimes et exercices physiques viennent rarement seuls à bout de l'obésité et des troubles métaboliques associés, on observe un intérêt grandissant pour de nouvelles interventions aussi bien pharmacologiques que cosmétiques qui visent les « désordres métaboliques ».

Le tissu adipeux hypodermique constitue le plus grand réservoir énergétique de l'organisme. Il est capable de stocker des lipides sous forme de triglycérides par un processus de lipogenèse puis de les libérer sous forme d'acides gras et de glycérol par un processus de lipolyse. C'est l'équilibre entre ces deux voies métaboliques qui conditionne le taux de graisse dans les tissus. Les réserves lipidiques de l'organisme se renouvellent en permanence et sont en relation étroite avec les apports nutritionnels et les besoins énergétiques de l'organisme. Si un déséquilibre s'installe dans l'organisme entre les processus de lipogenèse et de lipolyse, le volume et le nombre des adipocytes peuvent augmenter et on parle d'hypertrophie et d'hyperplasie adipocytaires. Le développement excessif de la masse adipeuse peut alors se traduire par des modifications de l'aspect de la peau, qui peut évoluer vers une surcharge pondérale de l'individu pouvant aller jusqu'à l'obésité.

La cellulite, l'aspect inesthétique en « peau d'orange » de la peau résulte de cette hypertrophie du tissu adipeux, d'un stockage de graisse sous-cutanée accompagné d'une rétention d'eau et d'un vieillissement du tissu conjonctif.

La cellulite apparaît quand il y a rupture d'équilibre entre lipogenèse et lipolyse. Lorsqu'ils prennent du volume, les adipocytes sont «poussés» vers la surface cutanée, expliquant ces irrégularités en « bosses et creux ». L'accentuation du tissu adipeux est localisée, en particulier chez la femme, au niveau des hanches, des cuisses, des fesses, des genoux et des avant-bras. Il faut souligner qu'une accumulation excessive de graisses dans les adipocytes qui deviennent hypertrophiés entraîne souvent une inflammation qui rend la cellulite douloureuse au toucher. Ceci est dû aux troubles de la micro circulation avec mauvaise oxygénation tissulaire et à la stase lymphatique avec rétention d'eau.

En général, l'obésité de l'homme est étroitement associée à un état inflammatoire chronique, qui concerne également le tissu adipeux avec une production accrue d'adipokines pro-inflammatoires *(*Lago F et al. Adipokines as emerging mediators of immune response and inflammation, Nat Clin Pract Rheumatol. 2007;3:716*.* Conde J et al. Adipokines: Biofactors from white adipose tissue. A complex hub among inflammation, metabolism, and immunity. Biofactors. 2011 Oct 28. doi: 10.1002*).*

Il a été récemment montré que Sirt1 en plus de son implication dans le métabolisme énergétique joue un rôle important dans la régulation de la réponse inflammatoire. En effet, l'activation de Sirt1 induit une réduction de la réponse inflammatoire des adipocytes *(*Yoshizaki T et al. SIRT1 exerts anti-inflammatory effects and improves insulin sensitivity in adipocytes. Mol. Cell. Biol. 2009;29:1363*)* ainsi que des macrophages qui représentent un des composants du tissu adipeux *(*Yoshizaki T et al, SIRT1 inhibits inflammatory pathways in macrophages and modulates insulin sensitivity. Am J Physiol Endocrinol Metab, 2010;298:E419*).* De plus, l'activation de Sirt1 réduit également l'état inflammatoire dans les fibroblastes (Zhu X et al. Activation of Sirt1 by resveratrol inhibits TNF- α induced inflammation in fibroblasts. PLoS One. 2011;6:e27081).

Plusieurs voies de recherche se développent afin de trouver une manière efficace de lutter contre la cellulite et les surcharges graisseuses en général. De nombreuses méthodes ont été employées, comme les techniques médicochirurgicales telles que la lipoplastie ou la liposuccion, le drainage lymphatique, la mésothérapie, la lipocavitation par les ultrasons... Cependant ces techniques, bien qu'efficaces, sont lourdes et contraignantes.

Ainsi il subsiste un besoin constant d'agir d'une manière non invasive sur les mécanismes de formation et/ou de destruction des surcharges graisseuses sous-cutanées et de la cellulite par des actifs cosmétiques capables de prévenir l'apparition de cellulite en inhibant la lipogenèse ou bien en favorisant la lipolyse.

Les nouveaux produits amincissants s'articulent, entre autres, autour de Sirt1 dont l'activité enzymatique n'aide pas seulement à prolonger la jeunesse de nos cellules mais également participe activement à la régulation du métabolisme lipidique *(*Lomb DJ et al. Sirtuins regulate key aspects of lipid metabolism. Biochim Biophys Acta. 2010;1804:1652*;* Schug TT et al. Sirtuin 1 in lipid metabolism and obesity. Ann Med. 2011;43:198*).* De plus, il est connu que l'expression de Sirt1 décroît progressivement avec l'âge et cela pourrait contribuer, au moins en partie, à la prévalence de l'obésité à l'âge adulte.

Les études récentes ont révélé que l'activation de Sirt1 atténue l'adipogenèse (la fabrication du tissu adipeux et le stockage de lipides par les adipocytes) *(*Bäckesjö CM et al. Activation of Sirt1 decreases adipocyte formation during osteoblast differentiation of mesenchymal stem cells. Cells Tissues Organs. 2009;189:93 *;* Fischer-Posovszky P et al. Resveratrol regulates human adipocyte number and function in a Sirt1-dependent manner. Am J Clin Nutr. 2010;92:5*).* Ainsi, l'utilisation des activateurs de Sirt1 comme moyen de prévention ou de traitement local des dysfonctionnements métaboliques associés à la présence de surcharges adipeuses et à l'état inflammatoire, peut-être considérée comme une nouvelle arme contre l'obésité.

Il a maintenant été mis en évidence que les dérivés de stéroïdes de formule (I) étaient capables d'augmenter l'activité enzymatique de Sirt1. Ces composés sont ainsi particulièrement utiles dans ou pour la préparation de compositions pharmaceutiques ou cosmétiques, pour traiter et/ou prévenir l'obésité ou l'inflammation, pour lutter contre les signes de vieillissement cutané ainsi que pour réduire les surcharges graisseuses sous-cutanées et la cellulite et/ou l'état inflammatoire associé.

Les différents aspects de l'invention sont définis dans les revendications.

Ainsi, selon un premier aspect, l'invention a pour objet un composé de formule (I) : 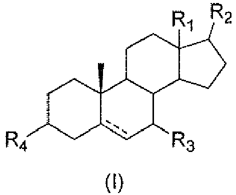 Dans laquelle :
R₁, R₂ représentent chacun indépendamment un atome d'hydrogène, un groupe C₁-C₆ alkyle, ou (CH₂)OR₅ ou (CH₃)CHNR₆R₇,
   ou bien
R₁ et R₂ forment ensemble un hétérocycle éventuellement substitué par un ou plusieurs groupes C₁-C₆ alkyle, =O, -C(=O)CH₃ ;
R₃ représente un atome d'hydrogène, un groupe OH, C₁-C₆ alkyle ou =O ;
R₄ représente un atome d'hydrogène, un groupe OH ou un groupe NR₈R₉ ;
- représente une liaison simple ou une double liaison ;
R₅ représente un atome d'hydrogène, un groupe (C₁-C₆) alkyle, ou un groupe (C₁-C₆)alkylcarbonyle;
R₆, R₇, R₈, R₉, représentent chacun indépendamment un atome d'hydrogène, un groupe C₁-C₆ alkyle, ou un groupe (C₁-C₆) alkylcarbonyle ;
ou une forme tautomère, énantiomère, diastéréoisomère, épimère ou un sel pharmaceutiquement acceptable de celui-ci,
   pour son utilisation dans le traitement et/ou la prévention de l'obésité, et/ou de l'inflammation liée à l'obésité et/ou à la cellulite via une augmentation de l'activité Sirt1.

Selon la présente invention, les radicaux alkyle représentent des radicaux hydrocarbonés saturés, en chaîne droite ou ramifiée, de 1 à 6 atomes de carbone, de préférence de 1 à 4 atomes de carbone.

On peut notamment citer, lorsqu'ils sont linéaires, les radicaux méthyle, éthyle, propyle, butyle, pentyle, et hexyle.

On peut notamment citer, lorsqu'ils sont ramifiés ou substitués par un ou plusieurs radicaux alkyles, les radicaux isopropyle, tert-butyl, 2-méthylbutyle, 2-méthylpentyle, et 1-méthylpentyle.

Les radicaux alkylcarbonyles selon la présente invention sont des radicaux de formule alkyl-C(=O)-, l'alkyle étant tel que défini précédemment.

Les radicaux hétérocycles désignent les systèmes mono ou bicycliques, saturés ou partiellement insaturés, non aromatiques, de 5 à 10 atomes de carbone comprenant un ou plusieurs hétéroatomes choisis parmi N, O ou S. De préférence, l'hétérocycle est un système monocyclique de 5 à 7 chaînons, notamment de 5 à 6 chaînons.

Parmi les composés de formule (I), un sous-groupe de l'invention est constitué par des composés pour lesquels R₁ est CH₃ ou CH₂OC(=O)CH₃.

Parmi les composés de formule (I), un autre sous-groupe de l'invention est constitué par des composés pour lesquels R₂ est (CH₃)CHN(CH₃)₂ ou (CH₃)CH-NHC(=O)CH₃.

Parmi les composés de formule (I), un autre sous-groupe de l'invention est constitué par des composés pour lesquels R₁ et R₂ forment ensemble un hétérocycle choisi parmi :

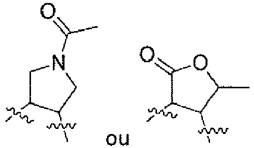

Parmi les composés de formule (I), un sous-groupe de l'invention est constitué par des composés pour lesquels R₃ est un groupe H, OH ou =O.

Parmi les composés de formule (I), un sous-groupe de l'invention est constitué par des composés pour lesquels R₄ est OH, N(CH₃)(C(=O)CH₃) ou NH(C(=O)CH₃).

Parmi les composés de formule (I), un autre sous-groupe de l'invention est constitué par les composés pour lesquels R₄ représente un atome d'hydrogène, ou un groupe NR₈R₉, notamment N(CH₃)(C(=O)CH₃) ou NH(C(=O)CH₃).

Selon un mode de réalisation, le composé de formule (I) est choisi parmi :
- 20-(diméthylamino)-, (3**β**,5**α**,20S)-pregnan-3-ol, (ci-après dénommé composé **C1**) ;
- N-[(3 **β**, 5 **α**, 20S)-20-(diméthylamino)pregnan-3-yl]-N-méthyl-acétamide, (ci-après dénommé composé **C2**) ;
- Acétate de 3 **β**, 20 **α**-diacétamido-pregn-5-en-18-ol, (ci-après dénommé composé **C3**) ;
- Acide 3-(acétylméthylamino)-7,20-dihydroxy-, γ-lactone, (3 **β**, 7 **β** ,20S)-pregn-5-èn-18-oïque, (ci-après dénommé composé **C4**) ;
- Acide 20 **α**-hydroxy-3 **β**-(N-méthylacétamido)-7-oxo-, γ-lactone pregn-5-èn-18-oïque, (ci-après dénommé composé **C5**) ;
- N-acétyl-3 **β**-(N-méthylacétamido)-norcon-5-énine, (ci-après dénommé composé **C6**) ;

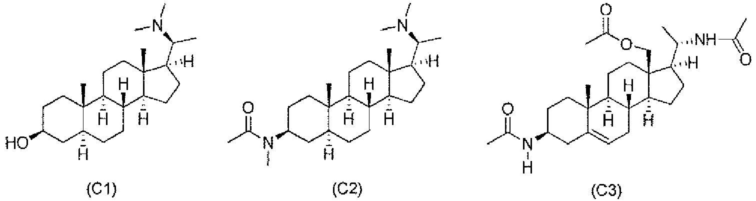

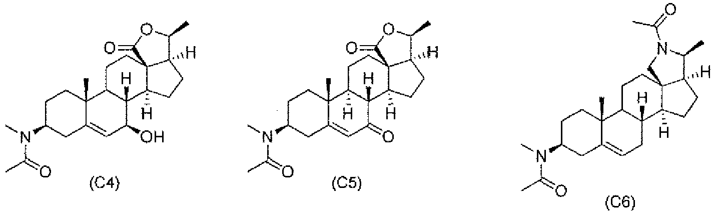

De préférence, le composé de formule (I) est choisi parmi les composés C2, C3, C4, C5 et C6.

Les composés de formule générale (I) peuvent être préparés par application ou adaptation de toute méthode connue en soi de et/ou à la portée de l'homme du métier, notamment celles décrites par Larock dans Comprehensive Organic Transformations, VCH Pub., 1989, ou par application ou adaptation des procédés décrits dans la littérature (voir notamment pour le composé **C1** : Khuong-Huu-Laine, et al., Annales Pharmaceutiques Francaises (1965), 23(6), 395-409 ; Vetter, W. et al. Bulletin de la Société Chimique de France (1963), 1324-30 ; Janot, M. M. et al., Bulletin de la Société Chimique de France (1962), 111-18 ; pour le composé **C2**, Khuong-Huu-Qui et al., Bulletin de la Société Chimique de France (1965), (10), 3035-40. ; pour le composé **C3**, Gods, D. D. et al., Tetrahedron (1963), 19 783-8 ; pour le composé **C4**, Husson, H. P. et al., Bulletin de la Société Chimique de France (1969), 9 3162-6 ; Fernandes, L. et al., Comptes Rendus des Séances de l'Académie des Sciences, Série C: Sciences Chimiques (1967), 264(26), 2165-8 ; pour le composé **C5**, Husson, H. P. et al.; Bulletin de la Société Chimique de France (1969), 9, 3162-6 ; Fernandes, L. et al.; Comptes Rendus des Séances de l'Académie des Sciences, Série C: Sciences Chimiques (1967), 264(26), 2165-8 ; pour le composé **C6**, Janot, M. M. et al. Annales Pharmaceutiques Françaises (1967), 25(11), 733-48.)

Les composés de formule (I) sont mis à disposition dans des compositions pharmaceutiques et/ou cosmétiques.

Les compositions pharmaceutiques et/ou cosmétiques sont notamment des compositions topiques ou aptes à être injectées (mésothérapie). Les compositions pharmaceutiques sont ainsi notamment des compositions dermatologiques. Ces compositions contiennent généralement un milieu physiologiquement acceptable. Par milieu physiologiquement acceptable, on entend un milieu compatible avec la peau, les muqueuses, les ongles et/ou les cheveux.

Les composés de formule (I) peuvent être présents dans une composition en une quantité allant de 0,001 à 30%, notamment de 0,01 à 10%, et plus particulièrement de 0,1 à 5% en poids du poids total de la composition.

Les compositions contenant un composé de formule (I) peuvent se présenter en particulier sous toutes les formes galéniques normalement appropriées pour une application topique, par exemple sous forme d'une solution aqueuse alcoolique, hydroalcoolique ou huileuse, d'un gel aqueux ou huileux, d'une composition solide anhydre, d'une dispersion du type lotion ou sérum, d'une émulsion du type eau-dans-huile (E/H), huile-dans-eau (H/E) ou multiple (E/H/E ou H/E/H), d'une microémulsion ou d'une dispersion de vésicules de type ionique et/ou non ionique. Ces compositions sont préparées selon les méthodes usuelles.

Ces compositions peuvent comprendre au moins un additif choisi parmi les corps gras, les conservateurs, les gélifiants, les parfums, les silicones, les tensioactifs, l'eau, les antioxydants, les charges, les filtres, les actifs physiologiquement acceptables tels que les hydratants, les vitamines, les actifs anti-vieillissement autres que les composés de formule (I), et leurs mélanges.

Les corps gras peuvent être choisis parmi les huiles telles que l'huile de jojoba, l'huile de vaseline, le palmitate d'isopropyle, les silicones (cyclopentadimethylsiloxane), les cires telles que la cire de sipol, la cire de camauba, les acides gras, les alcools gras (alcool stéarylique) et leurs mélanges.

Les quantités des différents constituants des compositions sont celles classiquement utilisées dans les domaines considérés.

Ces compositions constituent notamment des crèmes de traitement ou de soin pour le visage, pour les mains ou pour le corps, (par exemple crèmes de jour, crèmes de nuit), des produits de maquillage comme des fonds de teint, des laits corporels de protection ou de soin, des crèmes amincissantes pour le corps, des lotions, gels ou mousses pour le soin de la peau.

Selon un autre aspect, l'invention a pour objet l'utilisation d'un composé de formule (I) tel que défini en revendication 8 pour améliorer l'éclat du teint et/ou pour lisser la peau du visage et/ou du corps et/ou pour traiter les rides et les ridules de la peau et/ou pour stimuler le processus de renouvellement épidermique.

Les compositions cosmétiques et/ou dermatologiques peuvent être également destinées à réduire la masse graisseuse, en particulier les amas-graisseux sous-cutanés, notamment la cellulite.

Par l'expression « réduction de la masse graisseuse », on entend la réduction des tissus adipeux et/ou la réduction de poids d'un individu. L'individu peut souffrir ou non d'obésité. Cette expression inclut également une réduction de la prise de poids et/ou de l'augmentation de la masse graisseuse.

Tel qu'on l'utilise ici, le terme « obésité » se réfère à un état caractérisé par une surcharge pondérale d'un individu par rapport à sa taille, comparativement à un état dit « normal ». De préférence, tel qu'employé ici, il est dit qu'un individu humain souffre d'obésité, ou est obèse, si son Indice de Masse Corporelle (IMC = poids (kg) / taille² (m²)) est d'au moins 30 kg/m². Lorsque l'IMC est supérieure à 35-40, l'obésité est dite sévère ou morbide. Toutefois, les composés de formule (I) peuvent être appliqués ou administrés à tout individu dont le poids, en particulier la masse graisseuse, nécessite d'être réduite, que l'individu soit obèse ou non. Lorsque l'individu humain n'est pas obèse, ou présente une obésité qui n'est associée à aucun trouble, pathologie, ou risque lié à l'obésité, tel qu'un individu ayant une IMC inférieure à 35-40, l'utilisation des composés de formule (I) pour réduire le poids, notamment la masse graisseuse ne devrait pas être considérée comme une méthode de traitement thérapeutique mais comme un procédé de traitement cosmétique.

De préférence, les compositions cosmétiques et/ou dermatologiques sont administrées à des individus ne souffrant pas de troubles liés à une surcharge pondérale ou à l'obésité.

Par « troubles liés à une surcharge pondérale ou à l'obésité », on entend ici des pathologies dont les étiologies comprennent une surcharge pondérale ou l'obésité. Parmi ces pathologies, on peut citer notamment le diabète de type II, l'apnée du sommeil, les maladies cardiaques, les maladies respiratoires et l'ostéoarthrite.

L'invention a également pour objet un procédé de traitement cosmétique comprenant l'application sur la peau à traiter, d'un composé de formule (I) tel que défini ci-dessus, dans un véhicule physiologiquement acceptable.

L'application sur la peau comprend notamment une administration par voie topique ou par injection, de préférence sous-cutanée (juste sous la peau, mésothérapie).

Le procédé de traitement cosmétique selon l'invention peut être destiné à prévenir et/ou traiter les signes cutanés du vieillissement, pour améliorer l'éclat du teint et/ou pour lisser la peau du visage et/ou du corps et/ou pour traiter les rides et les ridules de la peau et/ou pour stimuler le processus de renouvellement épidermique, ou encore via une augmentation de l'activité Sirt1 à réduire la masse graisseuse, en particulier les amas graisseux sous-cutanés, notamment la cellulite.

L'invention concerne également les composés de formule (I) pour leur utilisation en mésothérapie, notamment esthétique, en particulier dans une méthode de traitement cosmétique. L'invention concerne également l'utilisation *in vitro* d'un composé de formule (I) tel que défini ci-dessus à titre d'outil pharmacologique, en particulier pour cribler et/ou identifier d'autres molécules capables d'activer l'activité enzymatique de Sirt1.

D'autres caractéristiques et avantages de l'invention ressortiront mieux des exemples qui suivent, donnés à titre illustratif et non limitatif.

### Figures

Figure 1 : Effet des composés sur l'activité enzymatique de Sirt1. Les résultats sont exprimés en % d'augmentation par rapport au contrôle.
Figure 2 : Effet des composés (10 µM) sur le stockage de lipides par des adipocytes humains au jour 14 de leur différentiation (coloration des lipides par l'Oil Red O); agrandissement x20.
Figure 3 : Effet des composés (10 µM) sur le stockage de lipides par des adipocytes humains au jour 14 de leur différentiation (coloration des lipides par l'Oil Red O); agrandissement x40.
Figure 4 : Effet des composés (50 µM) sur le stockage de lipides par des adipocytes humains au jour 14 de leur différentiation (coloration des lipides par l'Oil Red O) ; agrandissement x20.
Figure 5 : Effet des composés (50 µM) sur le stockage de lipides par des adipocytes humains au jour 14 de leur différentiation (coloration des lipides par l'Oil Red O); agrandissement x40.
Figure 6 : Effet des composés sur le taux de triglycérides présents dans des adipocytes humains après 7 (A) ou 14 (B) jours de différentiation.
Figure 7 : Effet des composés (50 µM) sur la lipolyse (taux de glycérol secrété par les adipocytes humains matures traités pendant 72 heures).
Figure 8 : Effet des composés (50 µM) sur la production de TNF-alpha par des monocytes humains après stimulation par le LPS.
Figure 9 : Effet des composés sur le taux d'adipsine sécrétée dans les milieux de culture par des adipocytes humains au jour 7 (A) et au jour 14 (B) jours de différentiation.

### Exemples

### Exemple 1 : Etude de l'effet des composés sur l'activité enzymatique de Sirt1

L'activité enzymatique de Sirt1 a été évaluée à l'aide du kit «HTRF^{R} SIRT1 Assay » vendu par la Société Cisbio. Lors de ces expériences, l'enzyme purifiée a été incubée à température ambiante en présence du NAD (cofacteur) de son substrat acétylé et du composé à tester utilisé aux concentrations variant de 0,24 µM à 280 µM. « SIRT1 Activator 3 », un activateur commercial de Sirt1 a été utilisé comme un contrôle positif. Après 30 min d'incubation, la réaction enzymatique est arrêtée et la quantité de substrat acétylé restante est évaluée à l'aide d'un anticorps spécifique.

Une augmentation de l'activité enzymatique de Sirt1 en présence des composés décrits s'est traduite par une diminution de la quantité d'anticorps capable de se fixer sur le substrat et donc par une diminution de l'émission de fluorescence qui en résulte, comparé à la valeur obtenue avec l'enzyme seule. Les résultats sont exprimés en % d'augmentation de l'activité de Sirt1 par rapport à celle obtenue avec l'enzyme sans ajout des composés.

### Résultats

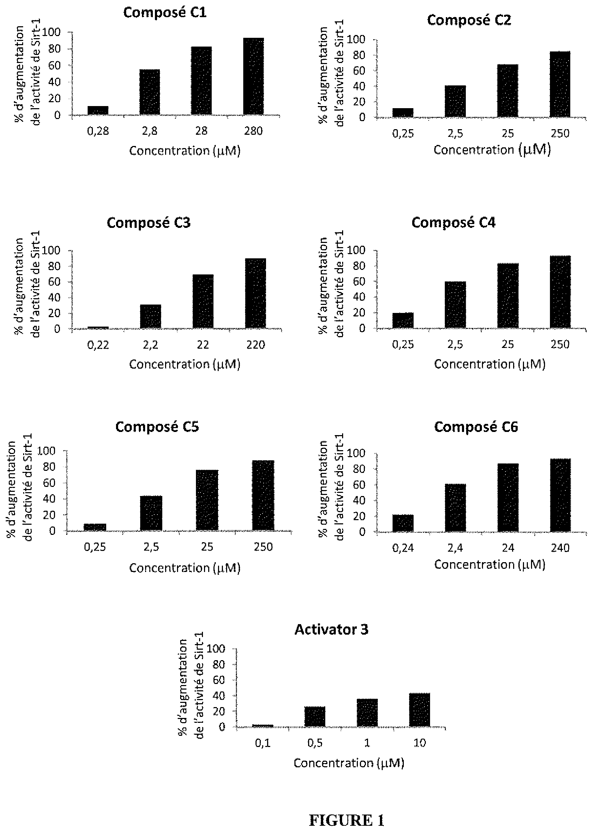

Les résultats obtenus montrent que tous les composés testés stimulent *in vitro* de façon dose- dépendante l'activité enzymatique de Sirt1 (Figure 1). Les composés C1, C4 et C6 sont les plus actifs en augmentant l'activité de Sirt1 de plus que 50% à la concentration d'environ 2,5 µM (1 µg/ml).

### Exemple 2 : Etude de l'activité antivieillissement d'une préparation contenant des composés stimulateurs de l'activité enzymatique de Sirt1

### Exemple 2a : Activité/expression de β-galactosidase

Le vieillissement cellulaire est accompagné en particulier d'une augmentation importante de la synthèse de β-galactosidase, phénomène caractéristique de la sénescence *in vitro* et *in vivo (*Itahana K et al. Methods to detect biomarkers of cellular senescence: the senescence-associated beta-galactosidase assay. Methods Moi Biol. 2007;371:21*).* Cette enzyme lysosomale hydrolyse une liaison osidique faisant intervenir un galactose en position β et elle constitue un marqueur du vieillissement cellulaire lorsque son activité est mesurée à pH 6. Son activité associée à la sénescence est détectable grâce à une coloration cytochimique qui est mise en évidence en utilisant le substrat chromogénique 5-bromo-4-chloro-3-indolyl β-galactopyranoside (X-gal) qui colore les cellules en bleu lorsqu'il est coupé par la β-galactosidase.

Cette étude a été réalisée sur des fibroblastes dermiques humains normaux (NHDF) fournis par la société Lonza et sur des cellules de la papille dermique de follicules capillaires (HFDPC) fournis par la société PromoCell. Les cellules ont été mises en culture dans des plaques de 24 puits (4 10⁴ cellules / puits) et après 24h, les cellules ont été traitées pendant 6 jours avec les composés C1 à C6 ajoutés individuellement dans les milieux de culture aux concentrations 10, 25 et 50 µM.

L'évaluation de l'activité enzymatique de la β-galactopyranoside a été évaluée à l'aide du kit « Senescence cells histochemical staining kit » de Sigma-Aldrich. Les cellules sont rincées deux fois avec du PBS puis elles sont incubées pendant 7 min dans la solution de fixation. Après trois rinçages au PBS, 200 µl de solution de coloration sont ajoutés aux cellules. Les cellules sont alors incubées durant 16 heures à 37°C sans CO₂. Les cellules sont ensuite rincées avec du PBS puis observées au microscope afin de déterminer la proportion de cellules positives pour l'activité β-galactosidase associée à la sénescence (devenues bleues) par rapport à l'ensemble de la population cellulaire.

Les résultats obtenus montrent que la proportion de cellules marquées en bleues dans lesquelles il existe une activité SA- β-galactosidase est moins importante chez les cellules traitées avec les produits aux concentrations testées par rapport aux cellules contrôles.

Il a donc été montré que par rapport au contrôle, les composés testés induisent une diminution significative de l'activité enzymatique de la β-galactosidase, ce qui résulte d'un ralentissement du vieillissement cellulaire.

### Exemple 3 : Etude de l'activité amincissante d'une préparation contenant des composés stimulateurs de l'activité enzymatique de Sirt1

Le tissu adipeux est constitué de cellules appelées adipocytes, capables de synthétiser des acides gras, de les estérifier en triglycérides, et ultérieurement d'hydrolyser ces lipides pour mettre les acides gras à la disposition des autres tissus. Son développement se déroule en trois étapes successives : prolifération cellulaire, différenciation et enfin grossissement des adipocytes.

Pour limiter une expansion des tissus adipeux, il est connu que l'on peut utiliser des substances qui peuvent agir sur les adipocytes, soit en stimulant la lipolyse des triglycérides intra-cellulaires et le re-largage extra-cellulaire des acides gras et du glycérol, soit au contraire en inhibant la lipogénèse de nouveaux triglycérides.

### Exemple 3a : Effet des composés stimulateurs de l'activité enzymatique de Sirt1 sur l'adipogenèse/lipagenèse

Les préadipocytes primaires sous-cutanés humains fournis par Lonza ont été mis en culture (10 000 cellules/puits dans des plaques de 96 puits) dans un milieu de croissance (PGM-2, Lonza, PT-9502) pendant une nuit. Au jour 1 (J1) les cellules ont été mises dans le milieu de différenciation en absence ou en présence du composé à tester. Chaque composé a été évalué à deux concentrations différentes (10 et 50 µM). Deux types de traitements ont été réalisés,

Pour le traitement court, les cellules ont été exposées pendant 7 jours de différenciation à la molécule testée qui a été ajoutée une fois à J1. Les cellules ont été photographiées et le taux de triglycérides intracellulaires a été mesuré à J7.

Pour le traitement long, les cellules on été traitées 2 fois avec les composés (J1 et J7). Les cellules colorées ont été photographiées et le taux de triglycérides intracellulaires a été mesuré à J14.
1) Coloration de triglycérides intracellulaires par le réactif « Oil Red O » de la société Sigma qui permet de visualiser des dépôts de lipides intracellulaires.
2) Quantification du taux de triglycérides intracellulaires à l'aide du test AdipoRed™ Assay Reagent (Lonza, PT7009). L'intensité de fluorescence (λem/ λex = 485/572 nm) est mesurée à l'aide d'un lecteur de fluorescence (PerkinElmer, Victor²).

### Résultats

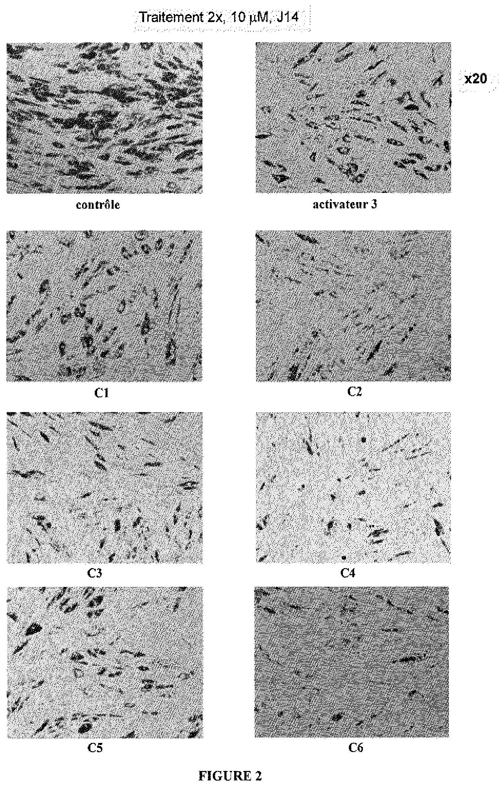

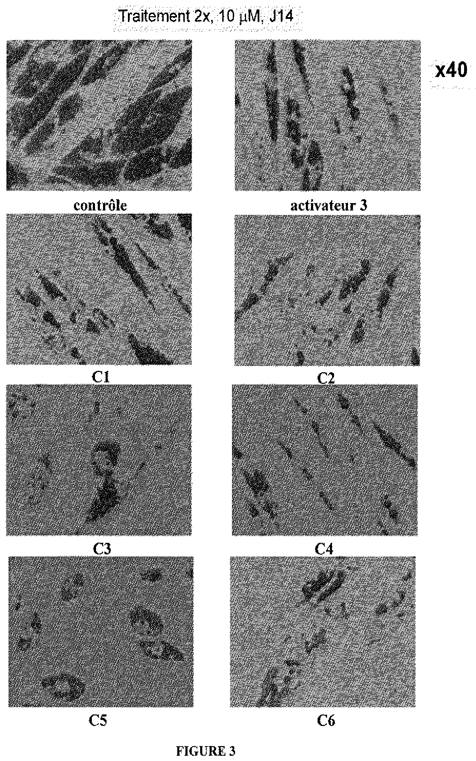

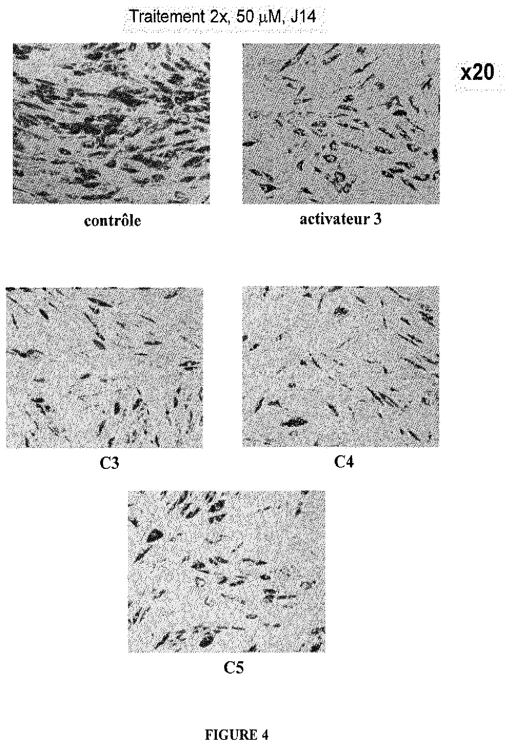

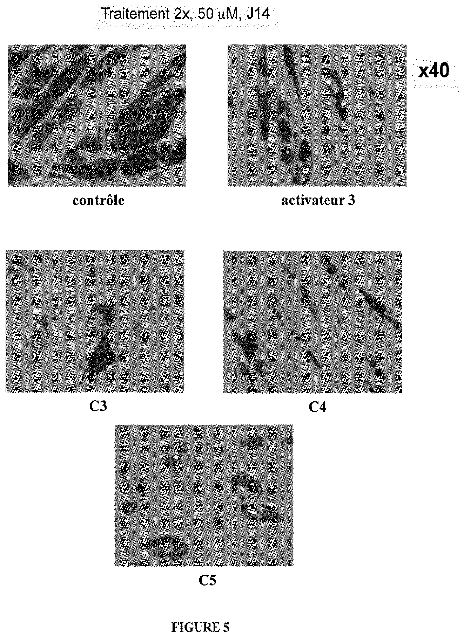

La coloration par l'Oil Red O des lipides neutres stockés dans les gouttelettes lipidiques des cellules adipocytaires montre une nette diminution de l'accumulation de lipides à J14 de leur différentiation suite au traitement avec les composés C1 à C6 (Figures 2 - 5).

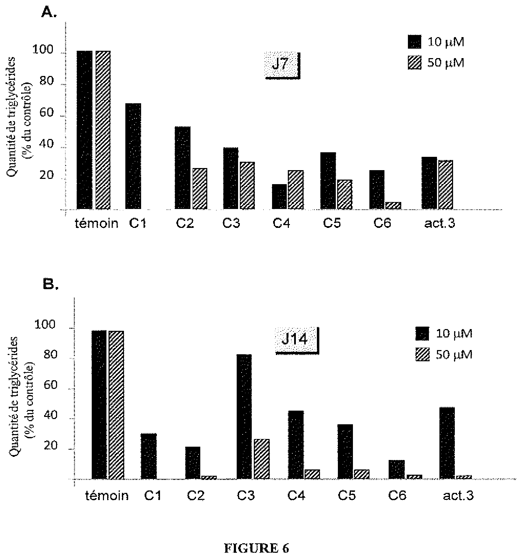

Le dosage de triglycérides présents dans les adipocytes témoins et traités avec les composés testés confirme ces observations microscopiques. Les résultats présentés dans la Figure 6 indiquent une diminution de la quantité des triglycérides au jour 7 (25 - 93%) (Figure 6A) et au jour 14 (16-98%) (Figure 6B) dans les adipocytes traités avec les composés C1 à C6.

L'ensemble de ces résultats montre que les composés C1 à C6 inhibent de façon significative la maturation des préadipocytes et qu'ils réduisent ainsi l'accumulation des lipides intracellulaires.

### Exemple 3b : Effet des composés stimulateurs de l'activité enzymatique de Sirt1 sur l'adipolyse

Les préadipocytes primaires sous-cutanés humains fournis par Lonza ont été mis en culture (10 000 cellules/puits dans des plaques de 96 puits) dans un milieu de croissance (PGM-2, Lonza, PT-9502) pendant une nuit. A J1, les cellules ont été mises dans le milieu de différenciation pendant 12 jours pour acquérir les caractéristiques des adipocytes matures. A J12, le milieu de culture a été supplémenté avec les composés testés à la concentration de 50 µM. Au J14, le taux de glycérol libéré par les adipocytes matures dans le milieu de culture est mesuré à l'aide du test « AdipoLyzeTM Détection Assay » de la société Lonza.

### Résultats

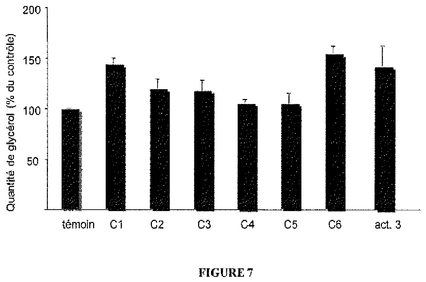

Le traitement des adipocytes matures pendant 72h avec les composés C1 à C6 (50 µM) montre que quatre des composés testés stimulent la lipolyse, ce qui se traduit par une augmentation de la quantité de glycérol (C1 +44%, C2 +24%, C3 +18%, C4 +10%, C5 +14% et C6 +41%) secrété par les adipocytes dans les surnageants de culture (Figure 7).

### Exemple 4 : Evaluation d'un effet anti-inflammatoire des composés stimulateurs de l'activité enzymatique de Sirt1

Les cellules monocytaires humaines THP-1 ont été mises en culture (1 million de cellules/puit dans des plaques 24 puits) dans du milieu RPMI1640 complémenté avec 10% de sérum de veau fœtal. Les cellules ont ensuite été pré-incubées pendant une heure avec soit « SIRT1 Activator 3 », soit les composés à tester ou avec le solvant. Les cellules ont ensuite été stimulées pendant 5 heures avec l'endotoxine (lipopolysaccharide, LPS) à la concentration de 10 ng/ml et la quantité de TNF-alpha secrétée dans le surnageant a été dosée à l'aide d'un kit ELISA (RD systems). Les résultats sont exprimés en picogrammes de TNF-alpha/ml de surnageant de culture.

### Résultats

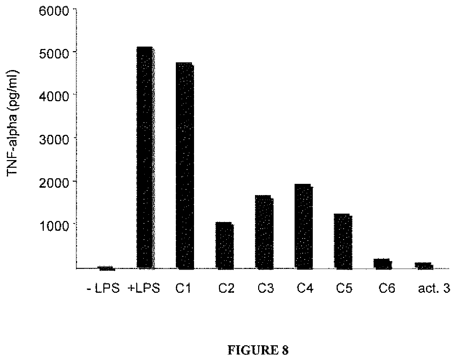

Le prétraitement des cellules THP-1 pendant 1h avec les composés C1 à C6 à la concentration de 100 µM réduit significativement (7%-96%) la production de TNF-alpha induite par le LPS. Les résultats présentés dans la Figure 8 montrent que deux des composés testés (C2 : -79% et C5 : -75%) entraînent une forte diminution de la sécrétion de TNF-alpha alors que le composé C6 (- 96%) inhibe presque totalement cette sécrétion pour atteindre un niveau équivalent à celui observé avec les cellules non stimulées par le LPS.

### Exemple 5 :

### Effet inhibiteur des composés stimulateurs de l'activité enzymatique de Sirt1 sur la production et/ou la sécrétion d'adipsine par des adipocytes

Le tissu adipeux est un organe endocrinien complexe qui communique avec le cerveau et les tissus périphériques en secrétant de nombreuses adipokines. L'adipsine, une des adipokines secrétée par le tissu adipeux, intervient de manière bien documentée dans l'obésité, le syndrome métabolique et le diabète de type 2.

L'adipsine est une enzyme (sérine protéase) nécessaire à la production d'une protéine stimulant l'acylation (ASP ; **A**cylation **S**timulating **P**rotein). Dans le tissu adipeux, l'ASP augmente la synthèse des triglycérides et diminue la lipolyse ainsi que la libération des acides gras libres (Havel PJ. Update on adipocyte hormones: regulation of energy balance and carbohydrate/lipid metabolism. Diabetes. 2004;53 Suppl 1:S143-51).

Chez les sujets obèses, le taux d'adipsine est augmenté et corrélé positivement au taux d'insuline. Ainsi, une réduction de la sécrétion d'adipsine devrait avoir pour conséquence une réduction du tissu adipeux.

Des préadipocytes primaires sous-cutanés humains fournis par Lonza (PT-5020) ont été mis en culture (30 000 cellules/puits dans des plaques de 48 puits) dans un milieu de croissance (PGM-2, Lonza, PT-9502) pendant une nuit. À J1, les cellules ont été mises dans le milieu de différenciation en absence ou en présence du composé à tester. « SIRT1 Activator 3 » a été utilisé comme un contrôle positif. Au J7 et au J14, le taux d'adipsine dans le milieu de culture a été mesuré à l'aide du système BioPlex (BioRad ; 171-A7002M). Chaque composé a été évalué à trois concentrations différentes (10, 25 et 50 µM).

### Résultats

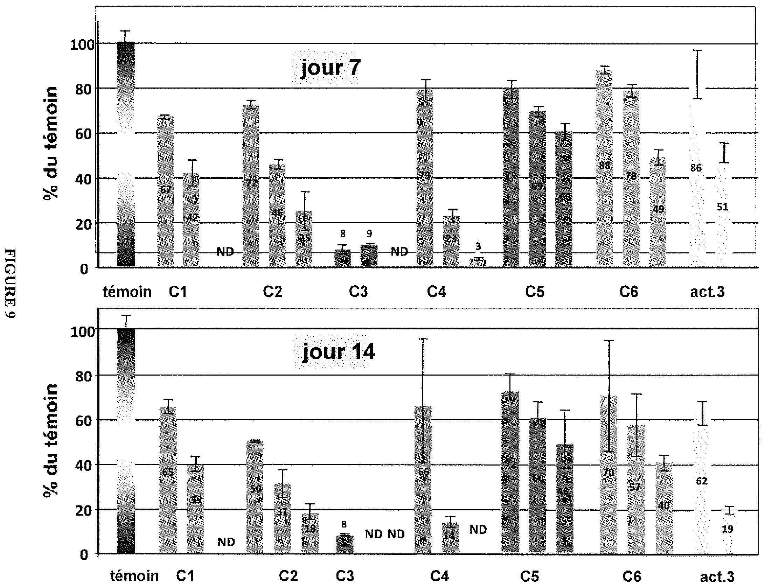

Le dosage de l'adipsine présente dans le milieu de culture des adipocytes témoins et traités avec les composés stimulateurs de l'activité enzymatique de Sirt1 montre leur effet inhibiteur sur la production et/ou la sécrétion d'adipsine par les cellules traitées. Les résultats présentés dans la Figure 9 indiquent une diminution systématique de la quantité d'adipsine au jour 7 (Figure 9A) et au jour 14 (Figure 9B) dans le milieu de culture des adipocytes traités avec les composés C1 à C6.

L'ensemble de ces résultats montre que les composés C1 à C6 inhibent de façon dose dépendante la concentration d'adipsine produite et/ou sécrétée par des cellules adipeuses.

## Revendications

1. Composé de formule (I) : 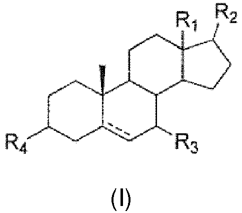 dans laquelle :
R₁, R₂ représentent chacun indépendamment un atome d'hydrogène, un groupe C₁-C₆ alkyle, ou (CH₂)OR₅ ou (CH₃)CHNR₆R₇,
ou bien
R₁ et R₂ forment ensemble un hétérocycle à 5 ou 6 chaînons, ledit hétérocycle éventuellement substitué par un ou plusieurs groupes C₁-C₆ alkyle, =O, -C(=O)CH₃ ;
R₃ représente un atome d'hydrogène, un groupe OH, C₁-C₆ alkyle ou =O ;
R₄ représente un atome d'hydrogène, un groupe OH ou un groupe NR₈R₉ ;
---- représente une liaison simple ou une double liaison ;
R₅ représente un atome d'hydrogène, un groupe (C₁-C₆) alkyle, ou un groupe (C₁-C₆) alkylcarbonyle ;
R₆, R₇, R₈, R₉, représentent chacun indépendamment un atome d'hydrogène, un groupe C₁-C₆ alkyle, ou un groupe (C₁-C₆) alkylcarbonyle ;
ou une forme tautomère, énantiomère, diastéréoisomère, épimère ou un sel pharmaceutiquement acceptable de celui-ci,
pour son utilisation dans le traitement et/ou la prévention de l'obésité, et/ou de l'inflammation liée à l'obésité et/ou à la cellulite via une augmentation de l'activité Sirt1.

2. Composé pour son utilisation selon la revendication 1, dans laquelle R₁ est CH₃ ou CH₂OC(=O)CH₃.

3. Composé pour son utilisation selon l'une quelconque des revendications 1 ou 2, dans laquelle R₂ est (CH₃)CHN(CH₃)₂ ou (CH₃)CH-NHC(=O)CH₃.

4. Composé pour son utilisation selon la revendication 1, dans laquelle R₁ et R₂ forment ensemble un hétérocycle :

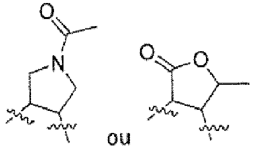

5. Composé pour son utilisation selon l'une quelconque des revendications précédentes, dans laquelle R₃ est un groupe H, OH ou =O.

6. Composé pour son utilisation selon l'une quelconque des revendications précédentes, dans laquelle R₄ est OH, N(CH₃)(C(=O)CH₃) ou NH(C(=O)CH₃).

7. Composé pour son utilisation selon la revendication 1, dans laquelle le composé de formule (I) est choisi parmi :
- 20-(diméthylamino)-, (3β,5α,20S)-pregnan-3-ol ;
- N-[(3β,5α,20S)-20-(diméthylamino)pregnan-3-yl]-N-méthyl-acétamide ;
- Acétate de 3β,20α-diacétamido-pregn-5-en-18-ol ;
- Acide 3-(acétylméthylamino)-7,20-dihydroxy-, γ-lactone, (3β, 7β, 20S)-pregn-5-èn-18-oïque ;
- Acide 20α-hydroxy-3β-(N-méthylacétamido)-7-oxo-, γ-lactone pregn-5-èn-18-oïque ;
- N-acétyl-3 β-(N-méthylacétamido)-norcon-5-énine.

8. Utilisation d'un composé de formule (I) : 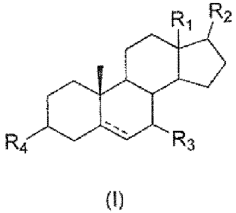 dans laquelle :
R₁, R₂ représentent chacun indépendamment un atome d'hydrogène, un groupe C₁-C₆ alkyle, ou (CH₂)OR₅ ou (CH₃)CHNR₆R₇,
ou bien
R₁ et R₂ forment ensemble un hétérocycle : 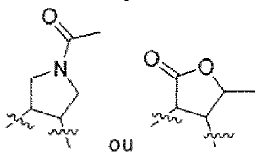
ledit hétérocycle éventuellement substitué par un ou plusieurs groupes C₁-C₆ alkyle, =O, -C(=O)CH₃ ;
R₃ représente un atome d'hydrogène, un groupe OH, C₁-C₆ alkyle ou =O ;
R₄ représente un atome d'hydrogène ou un groupe NR₈R₉ ;
---- représente une liaison simple ou une double liaison ;
R₅ représente un atome d'hydrogène, un groupe (C₁-C₆) alkyle, ou un groupe (C₁-C₆) alkylcarbonyle ;
R₆, R₇, R₈, R₉, représentent chacun indépendamment un atome d'hydrogène, un groupe C₁-C₆ alkyle, ou un groupe (C₁-C₆) alkylcarbonyle ;
ou une forme tautomère, énantiomère, diastéréoisomère, épimère ou un sel pharmaceutiquement acceptable de celui-ci,
pour améliorer l'éclat du teint et/ou pour lisser la peau du visage et/ou du corps et/ou pour traiter les rides et les ridules de la peau et/ou pour stimuler le processus de renouvellement épidermique.

9. Utilisation non-thérapeutique d'un composé de formule (I) telle que définie à la revendication 8, pour réduire la masse graisseuse chez un individu, en particulier les amas graisseux sous-cutanés, notamment la cellulite, via une augmentation de l'activité Sirt1.

10. Procédé de traitement cosmétique destiné à prévenir et/ou traiter les signes cutanés du vieillissement pour améliorer l'éclat du teint et/ou pour lisser la peau du visage et/ou du corps et/ou pour traiter les rides et les ridules de la peau et/ou pour stimuler le processus de renouvellement épidermique, comprenant l'application sur la peau à traiter, d'un composé de formule (I) tel que défini dans la revendication 8 dans un véhicule physiologiquement acceptable.

11. Procédé de traitement cosmétique non-thérapeutique destiné à réduire la masse graisseuse, en particulier les amas graisseux sous-cutanés, notamment la cellulite, via une augmentation de l'activité Sirt1, comprenant l'application sur la peau à traiter, d'un composé de formule (I) tel que défini dans la revendication 9 dans un véhicule physiologiquement acceptable.

12. Utilisation *in vitro* d'un composé de formule (I) tel que défini dans l'une quelconque des revendications 1 à 7 à titre d'outil pharmacologique, pour cibler et/ou identifier d'autres molécules capables d'activer l'activité enzymatique de Sirt1.

## Patentansprüche

1. Verbindung der Formel (I): 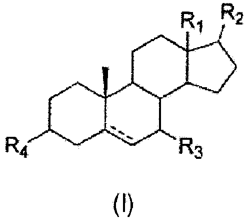 bei der:
R₁, R₂ jeweils unabhängig ein Wasserstoffatom, eine C₁-C₆-Alkylgruppe oder (CH₂)OR₅ oder (CH₃)CHNR₆R₇ darstellen,
oder auch
R₁ und R₂ zusammen einen Heterozyklus mit fünf oder sechs Kettengliedern bilden, wobei dieser Heterozyklus gegebenenfalls durch eine oder mehrere der Gruppen C₁-C₆-Alkyl, =O, -C(=O)CH₃ substituiert wird;
R₃ ein Wasserstoffatom, eine der -Gruppen OH, C₁-C₆-Alkyl oder =O darstellt;
R₄ ein Wasserstoffatom, eine OH-Gruppe oder eine NR₈R₉-Gruppe darstellt;
---- eine einfache Verbindung oder eine Doppelverbindung darstellt;
R₅ ein Wasserstoffatom, eine (C₁-C₆)Alkylgruppe oder eine (C₁-C₆)Alkylcarbonylgruppe darstellt;
R₆, R₇, R₈, R₉ jeweils unabhängig ein Wasserstoffatom, eine C₁-C₆-Alkylgruppe oder eine (C₁-C₆)Alkylcarbonylgruppe darstellen;
oder eine tautomere, enantiomere, diastereoisomere, epimere Form oder ein pharmazeutisch verträgliches Salz davon darstellen,
für ihre Verwendung in der Behandlung und/oder der Prävention der Fettleibigkeit und/oder der an die Fettleibigkeit gebundenen Entzündung und/oder der Cellulite über eine Verstärkung der Sirt1-Aktivität.

2. Verbindung für ihre Verwendung nach Anspruch 1, bei der R₁ CH₃ oder CH₂OC(=O)CH₃ ist.

3. Verbindung für ihre Verwendung nach einem beliebigen der Ansprüche 1 oder 2, bei der R₂ (CH₃)CHN(CH₃)₂ oder (CH₃)CH-NHC(=O)CH₃ ist.

4. Verbindung für ihre Verwendung nach Anspruch 1, bei der R₁ und R₂ zusammen einen Heterozyklus bilden:

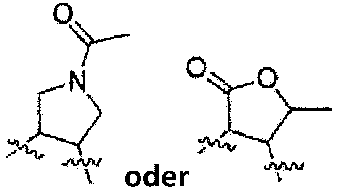

5. Verbindung für ihre Verwendung nach einem beliebigen der vorhergehenden Ansprüche, bei der R₃ eine Gruppe H, OH oder =O ist.

6. Verbindung für ihre Verwendung nach einem beliebigen der vorhergehenden Ansprüche, bei der R₄ OH, N(CH₃(C(=O)CH₃) oder NH(C(=O)CH₃) ist.

7. Verbindung für ihre Verwendung nach Anspruch 1, bei der die Verbindung der Formel (I) ausgewählt ist aus:
- 20-(dimethylamino)-, (3β,5α,20S)-pregnan-3-ol;
- N-[(3β,5α,20S)-20-(dimethylamino)pregnan-3-yl]-N-methyl-acetamid,
- Acetat von 3β,20α-diacetamido-pregn-5-en-18-ol;
- 3-(acetylmethylamino)-7,20-dihydroxy-, γ-lacton, (3β, 7β, 20S)-pregn-5-en-18-oische Säure;
- 20α-hydroxy-3β-(N-methylacetamido)-7-oxo-, γ-lacton, pregn-5-en-18-oische Säure;
- N-acetyl-3 β-(N-methylacetamido)-norcon-5-enin.

8. Verwendung einer Verbindung der Formel (I): 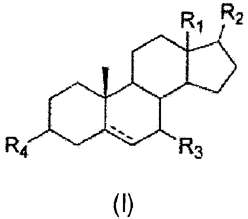 bei der:
R₁, R₂ jeweils unabhängig ein Wasserstoffatom, eine C₁-C₆-Alkylgruppe oder (CH₂)OR₅ oder (CH₃)CHNR₆R₇ darstellen,
oder auch
R₁ und R₂ einen Heterozyklus bilden: 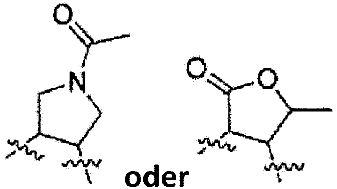
wobei der Heterozyklus gegebenenfalls substituiert ist durch eine oder mehrere der Gruppen C₁-C₆-Alkyl, =O, -C(=O)CH₃;
R₃ ein Wasserstoffatom, eine der Gruppen OH, C₁-C₆-Alkyl oder =O darstellt;
R₄ ein Wasserstoffatom, eine OH-Gruppe oder eine NR₈R₉-Gruppe darstellt;
---- eine einfache Verbindung oder eine Doppelverbindung darstellt;
R₅ ein Wasserstoffatom, eine (C₁-C₆)Alkylgruppe oder eine (C₁-C₆)Alkylcarbonylgruppe darstellt;
R₆, R₇, R₈, R₉ jeweils unabhängig ein Wasserstoffatom, eine C₁-C₆-Alkylgruppe oder eine (C₁-C₆)Alkylcarbonylgruppe darstellen;
oder eine tautomere, enantiomere, diastereoisomere, epimere Form oder ein pharmazeutisch verträgliches Salz davon darstellen,
zum Verbessern der Leuchtkraft der Haut und/oder zum Glätten der Haut des Gesichts und/oder des Körpers und/oder zum Behandeln von Falten und Fältchen der Haut und/oder zum Stimulieren des Prozesses der Epidermis-Erneuerung.

9. Nichttherapeutische Verwendung einer Verbindung der Formel (I), wie sie im Anspruch 8 definiert ist, zum Verringern der Fettmasse eines Individuums, insbesondere der Unterhautfettansammlung, insbesondere der Cellulitis, über die Verstärkung der Aktivität Sirt1.

10. Verfahren zur kosmetischen Behandlung, vorgesehen zum Verhindern und/oder zum Behandeln der kutanen Zeichen des Alterns zum Verbessern der Leuchtkraft der Haut und/oder zum Glätten der Haut des Gesichts und/oder des Körpers und/oder zum Behandeln der Falten und Fältchen der Haut und/oder zum Stimulieren des Prozesses der Epidermis-Erneuerung, umfassend die Anwendung einer Verbindung der Formel (I) wie im Anspruch 8 definiert in einem physiologisch akzeptablen Träger auf der zu behandelnden Haut.

11. Verfahren zur kosmetischen nichttherapeutischen Behandlung, die vorgesehen ist, die Fettmassen, insbesondere die subkutane Fettansammlung, nämlich die Cellulitis, über eine Verstärkung der Aktivität Sirt1 zu reduzieren, umfassend die Anwendung einer Verbindung der Formel (I), wie sie im Anspruch 9 definiert ist, in einem physiologisch akzeptablen Träger auf der zu behandelnden Haut.

12. In-vitro-Verwendung einer Verbindung der Formel (I), wie sie in einem beliebigen der Ansprüche 1 bis 7 definiert ist, als pharmakologisches Werkzeug, um andere Moleküle, die in der Lage sind, die Enzymtätigkeit des Sirt1 zu aktivieren, zu gewinnen und/oder zu identifizieren.

## Claims

1. Compound of formula (I): 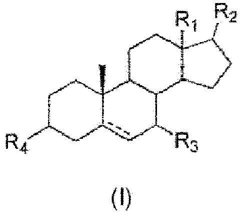 wherein:
R₁, R₂ each independently represents a hydrogen atom, a C₁-C₆-alkyl group, or
(CH₂)OR₅ or (CH₃)CHNR₆R₇,
or
R₁ and R₂ form together a heterocycle having 5 or 6 ring members, said heterocycle being optionally substituted by one or more C₁-C₆-alkyl, =OH, -C(=O)CH₃ groups;
R3 represents a hydrogen atom or an OH, C₁-C₆-alkyl or =O group;
R₄ represents a hydrogen atom, an OH group or a NR₈R₉ group;
---- represents a single bond or a double bond;
R₅ represents a hydrogen atom, a (C₁-C₆)alkyl group, or a (C₁-C₆)alkylcarbonyl group;
R₆, R₇, R₈, R₉ each independently represents a hydrogen atom, a C₁-C₆-alkyl group,
or a (C₁-C₆)alkylcarbonyl group;
or a tautomeric, enantiomeric, diastereoisomeric or epimeric form thereof or a pharmaceutically acceptable salt thereof,
for use in the treatment and/or prevention of obesity and/or of inflammation associated with obesity and/or with cellulite by increasing Sirt1 activity.

2. Compound for use according to claim 1, wherein R₁ is CH₃ or CH₂OC(=O)CH₃.

3. Compound for use according to either claim 1 or claim 2, wherein R₂ is (CH₃)CHN(CH₃)₂ or (CH₃)CH-NHC(=O)CH₃.

4. Compound for use according to claim 1, wherein R₁ and R₂ form together a heterocycle:

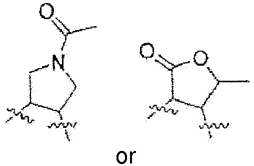

5. Compound for use according to any one of the preceding claims, wherein R₃ is a H, OH or =O group.

6. Compound for use according to any one of the preceding claims, wherein R₄ is OH, N(CH₃)(C(=O)CH₃) or NH(C(=O)CH₃).

7. Compound for use according to claim 1, wherein the compound of formula (I) is chosen from:
- 20-(dimethylamino)-(3β,5α,20S)-pregnan-3-ol;
- N-[(3β,5a,20S)-20-(dimethylamino)-pregnan-3-yl]-N-methyl-acetamide;
- 3β,20α-diacetamido-pregn-5-en-18-ol acetate;
- 3-(acetylmethylamino)-7,20-dihydroxy-γ-lactone-(3β,7β,20S)-pregn-5-en-18-oic acid;
- 20α-hydroxy-3β-(N-methylacetamido)-7-oxo-γ-lactone-pregn-5-en-18-oic acid;
- N-acetyl-3β-(N-methylacetamido)-norcon-5-enine.

8. Use of a compound of formula (I): 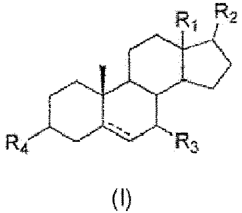 wherein:
R₁, R₂ each independently represents a hydrogen atom, a C₁-C₆-alkyl group, or
(CH₂)OR₅ or (CH₃)CHNR₆R₇,
or
R₁ and R₂ form together a heterocycle: 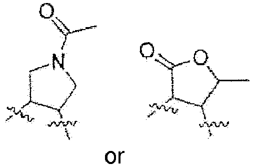
said heterocycle being optionally substituted by one or more C₁-C₆-alkyl, =O, -C(=O)CH₃ groups;
R3 represents a hydrogen atom or an OH, C₁-C₆-alkyl or =O group;
R₄ represents a hydrogen atom or a NR₈R₉ group;
---- represents a single bond or a double bond;
R₅ represents a hydrogen atom, a (C₁-C₆)alkyl group, or a (C₁-C₆)alkylcarbonyl group;
R₆, R₇, R₈, R₉ each independently represents a hydrogen atom, a C₁-C₆-alkyl group, or a (C₁-C₆)alkylcarbonyl group;
or a tautomeric, enantiomeric, diastereoisomeric or epimeric form thereof or a pharmaceutically acceptable salt thereof,
for improving the brightness of the complexion and/or for smoothing the skin of the face and/or of the body and/or for treating winkles and fine lines of the skin and/or for stimulating the process of epidermal renewal.

9. Non-therapeutic use of a compound of formula (I) as defined in claim 8 for reducing fat mass in an individual, in particular subcutaneous fat deposits, especially cellulite, by increasing Sirt1 activity.

10. Cosmetic treatment method for preventing and/or treating cutaneous signs of ageing in order to improve the brightness of the complexion and/or to smooth the skin of the face and/or of the body and/or to treat wrinkles and fine lines of the skin and/or to stimulate the process of epidermal renewal, which method comprises applying to the skin to be treated a compound of formula (I) as defined in claim 8 in a physiologically acceptable carrier.

11. Non-therapeutic cosmetic treatment method for reducing fat mass, in particular subcutaneous fat deposits, especially cellulite, by increasing Sirt1 activity, which method comprises applying to the skin to be treated a compound of formula (I) as defined in claim 9 in a physiologically acceptable carrier.

12. *In vitro* use of a compound of formula (I) as defined in any one of claims 1 to 7 as a pharmacological tool for targeting and/or identifying other molecules capable of activating the enzymatic activity of Sirt1.
